(19) Europäisches Patentamt
European Patent Office
Office européen des brevets

(11) **EP 2 719 325 A1**

(12) **EUROPEAN PATENT APPLICATION**
published in accordance with Art. 153(4) EPC

(43) Date of publication:
**16.04.2014 Bulletin 2014/16**

(51) Int Cl.:
**A61B 5/00** *(2006.01)* **A61B 5/055** *(2006.01)*
**A61B 6/00** *(2006.01)* **A61B 6/03** *(2006.01)*

(21) Application number: **12797081.2**

(22) Date of filing: **22.05.2012**

(86) International application number:
**PCT/JP2012/063097**

(87) International publication number:
**WO 2012/169344 (13.12.2012 Gazette 2012/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2011 JP 2011130254**

(71) Applicant: **Hitachi Medical Corporation Tokyo 101-0021 (JP)**

(72) Inventors:
• **BAN, Hideyuki**
  **Chiyoda-ku, Tokyo 100-8280 (JP)**
• **KURIHARA, Tsuneya**
  **Chiyoda-ku, Tokyo 100-8280 (JP)**

(74) Representative: **Strehl Schübel-Hopf & Partner Maximilianstrasse 54 80538 München (DE)**

(54) **IMAGE DIAGNOSIS ASSISTING APPARATUS, AND METHOD**

(57)    Improvement is made in efficiency of positioning between images when comparing a plurality of images to perform a radiographic image interpretation in an image diagnosis assisting apparatus. An image diagnosis assisting apparatus, which assists an image diagnosis by use of registration among a plurality of images, comprises: a radiographic image interpretation terminal (104) for executing the registration; and storage devices (101, 103) for storing model images and the like to be used for the registration. The radiographic image interpretation terminal (104) executes the registration among the plurality of images by use of a positioning reference area, which was defined beforehand in a model image selected on the basis of both a purpose of checking the plurality of images and a part of interest and also by use of parameters of the registration determined on the basis of a manipulation of checking the plurality of images.

FIG. 1A

EP 2 719 325 A1

**Description**

TECHNICAL FIELD

The present invention relates to an image diagnosis assisting apparatus, and specifically to a technology to improve efficiency of an alignment process between images when interpreting a plurality of images by comparison.

BACKGROUND ART

In recent image diagnoses, a plurality of images are often compared for interpretation, including a differential diagnosis determining whether a tumor mass is benign or malignant by comparing a plurality of images taken at different date and time such as during a follow-up or by comparing a plurality of images using different test equipments or different imaging techniques. In such a case, an organ in the image is often not displayed at the same position among the plurality of images to be compared due to a body motion caused by respiration or due to postural change at the time of taking the images. Therefore, it is desirable that, for executing an efficient diagnosis, an alignment between the images by moving, scaling, rotating, or deforming one of the images as needed, namely a registration process, is executed so that target sites in the plurality of images taken in advance are displayed at the same position.

As a conventional technology, for example, Non-patent Literature 1 discloses a technique of maximizing a mutual information amount which represents statistical dependency of corresponding pixel values between the images, as a registration process.

As another conventional technology, as in Patent Literature 1 for example, a technique of facilitating comparison by precisely aligning not the whole image but a display position of the target site by extracting a divergence of a bronchus from a plurality of images by image recognition and precisely matching the diverging position is disclosed.

Citation List

Patent Literature

Patent Literature 1: Japanese Patent Laid-open No. 2009-160045

Non-Patent Literature

Non-patent Literature 1: Journal of Institute of Electronics, Information and Communication Engineers D-II, Vol. J87-D-II, No. 10, pp.1887-1920, October 2004

SUMMARY OF INVENTION

TECHNICAL PROBLEM

Medical images encompass various test equipments (modalities) and imaging techniques depending on the test purpose, as well as images focusing on various sites. Thus, in order to execute the registration process using the aforementioned conventional technologies, there is a problem that parameters related to the process need to be adjusted according to the image to be aligned.

There is another problem that optimization of a recognition algorithm with respect to each site is required in order to extract the target site based on the image recognition using the aforementioned conventional technologies. There is also a problem that it is difficult to cope with a case of deformation or loss of the site due to an individual difference or a surgery.

An object of the present invention is to provide an image diagnosis assisting apparatus and a method capable of solving the aforementioned problems and improving efficiency of the alignment process between images when interpreting a plurality of images by comparison.

SOLUTION TO PROBLEM

To achieve the above object, the present invention provides an image diagnosis assisting apparatus that assists an image diagnosis by a registration process between a plurality of images, the image diagnosis assisting apparatus including a processing unit executing the registration process and a storage unit storing therein a parameter used for the registration process corresponding to a test technique, wherein the processing unit executes the registration process between the plurality of images using the parameter of the registration process selected based on the test technique for the plurality of images.

To achieve the above object, present invention further provides a method of operating an image diagnosis assisting apparatus using a terminal that assists an image diagnosis by a registration process between a plurality of images, wherein the terminal selects a model image based on a test purpose and a target site of the plurality of images, and the registration process between the plurality of images is executed using an alignment reference area preset to the selected model image and a parameter of the registration process determined based on a test technique for the plurality of images.

ADVANTAGEOUS EFFECTS OF INVENTION

The present invention enables an improvement of efficiency of an alignment process between images when interpreting a plurality of images by comparison.

BRIEF DESCRIPTION OF DRAWINGS

[Figure 1A]

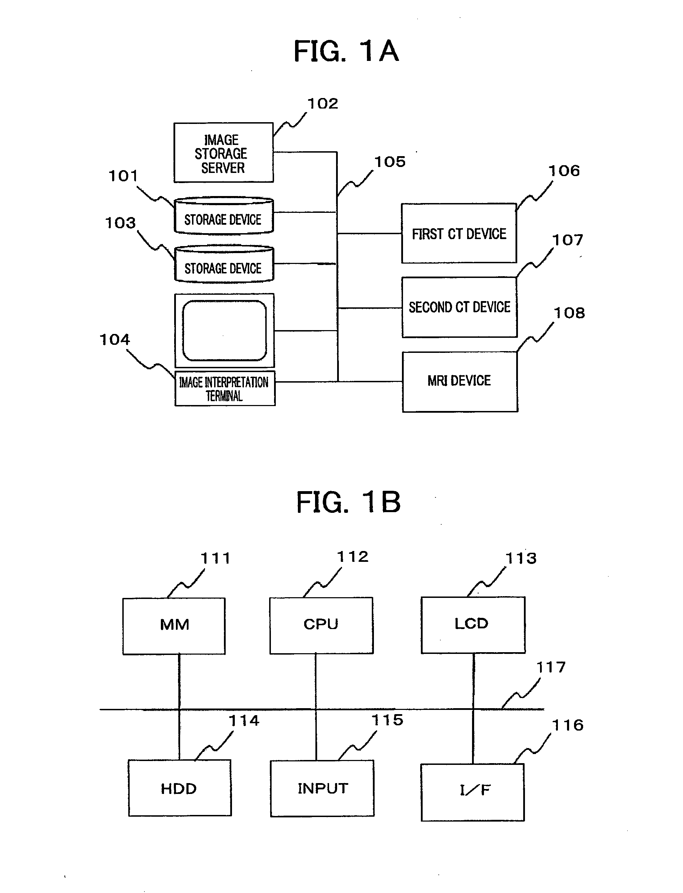

Figure 1A is a configuration diagram showing an example of an image diagnosis assisting system according to a first embodiment;
[Figure 1B]
Figure 1B is a block diagram showing an example of an internal configuration of the image diagnosis assisting system according to the first embodiment;
[Figure 2]

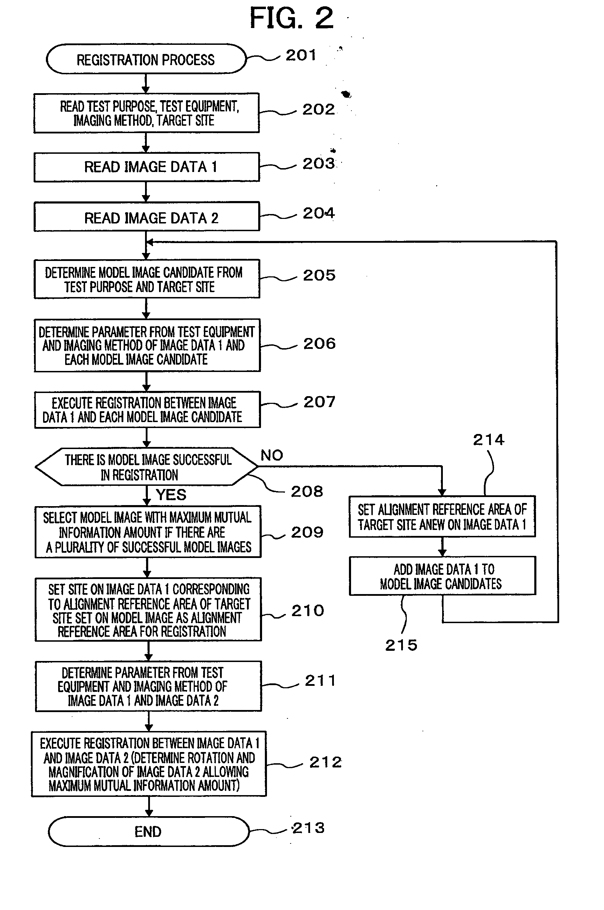

Figure 2 is a flow chart showing a processing procedure of a registration process according to the first embodiment;
[Figure 3]

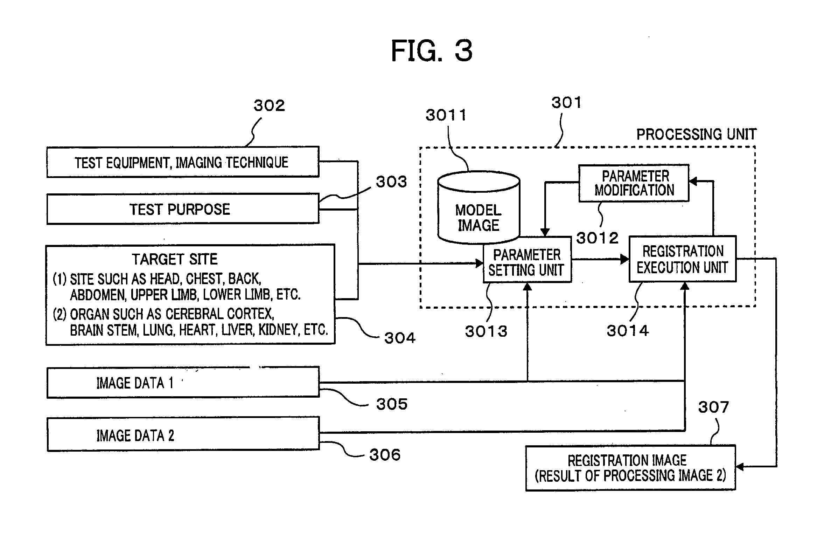

Figure 3 is a diagram illustrating an outline of the registration process according to the first embodiment;
[Figure 4]

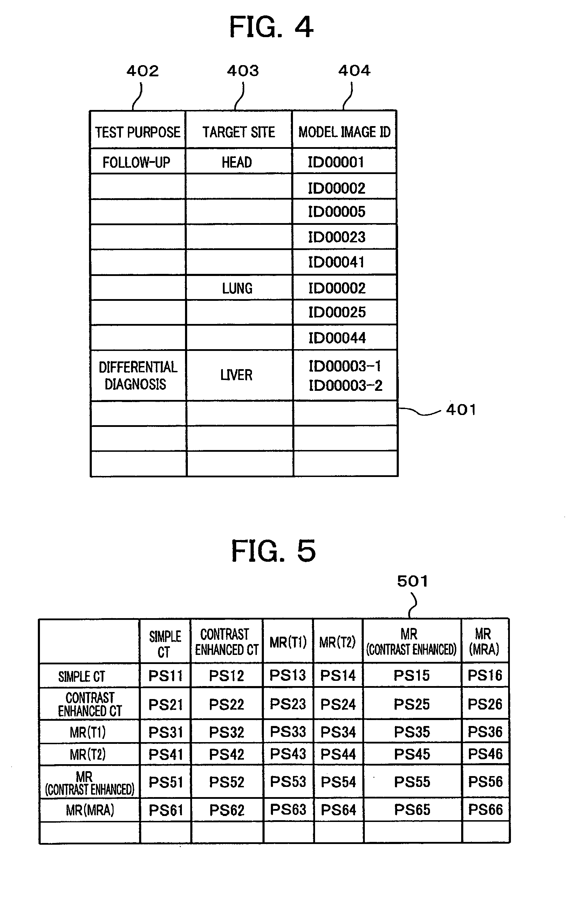

Figure 4 is a diagram showing an example of a model image candidate table according to the first embodiment;
[Figure 5]
Figure 5 is a diagram showing an example of a parameter set (PS) setting table according to the first embodiment;
[Figure 6]

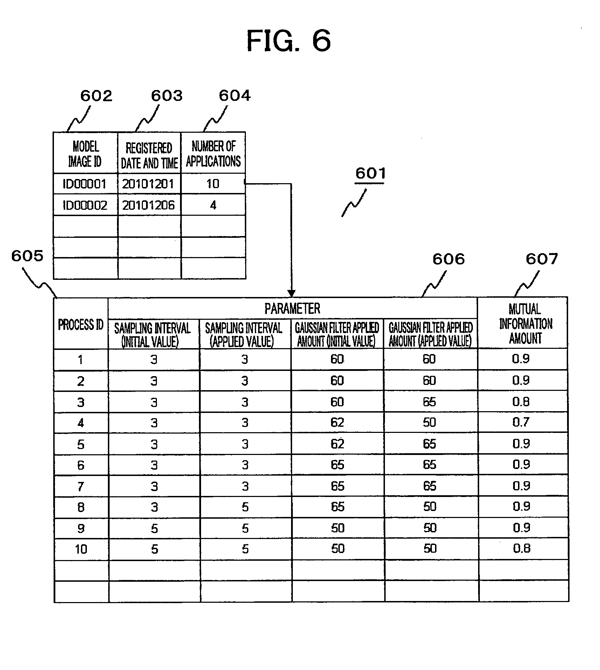

Figure 6 is a diagram showing an example of an execution result accumulation table according to the first embodiment;
[Figure 7]

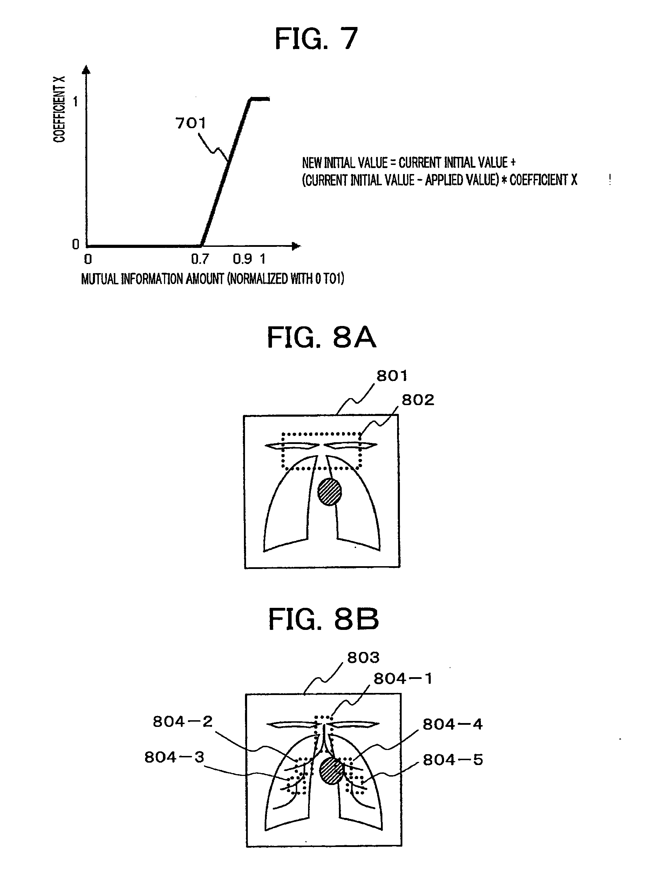

Figure 7 is a graphic chart illustrating an example of a parameter modification in the registration process according to the first embodiment;
[Figure 8A]
Figure 8A is a diagram showing an example of the model image according to the first embodiment;
[Figure 8B]
Figure 8B is a diagram showing another example of the model image according to the first embodiment;
[Figure 8C]

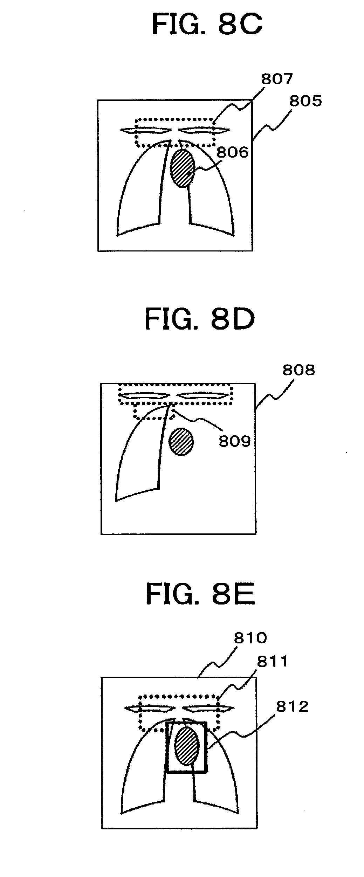

Figure 8C is a diagram showing an example of the model image according to a second embodiment;
[Figure 8D]
Figure 8D is a diagram showing another example of the model image according to the second embodiment;
[Figure 8E]
Figure 8E is a diagram showing another example of the model image according to the second embodiment;
[Figure 9]

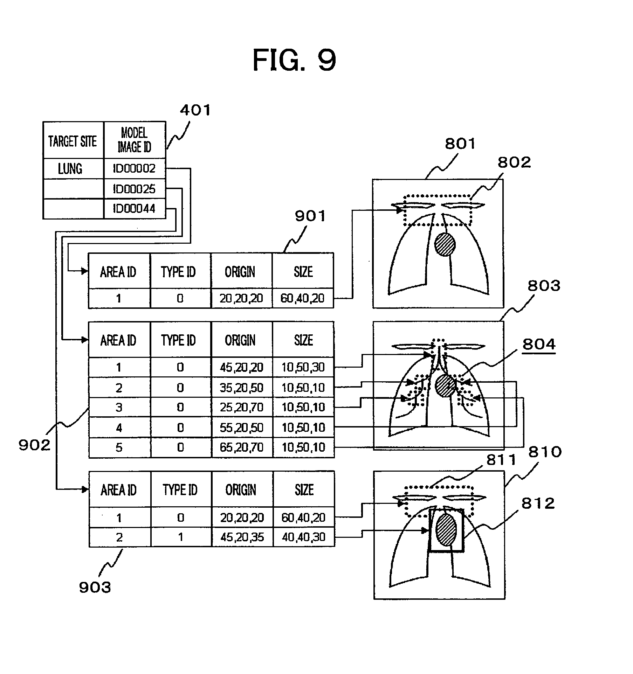

Figure 9 is a diagram showing another example of data of the model image stored in a storage device according to the first embodiment;
[Figure 10]

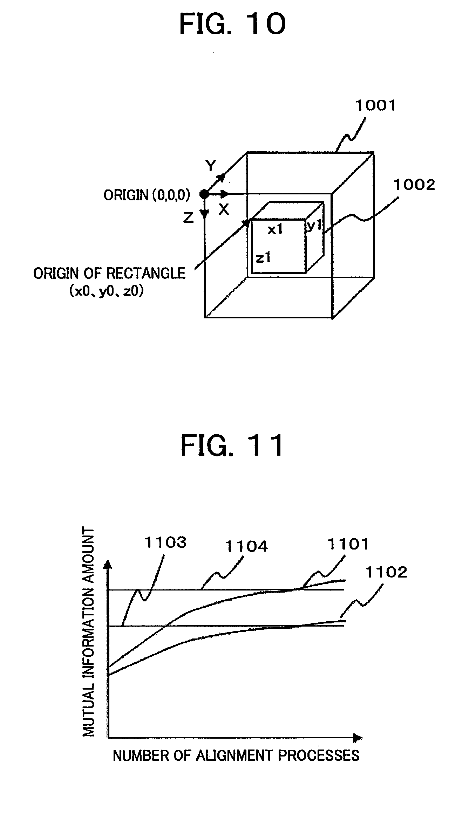

Figure 10 is a diagram illustrating a three-dimensional data management method for a target site and a model image according to each embodiment;
[Figure 11]
Figure 11 is a graphic chart illustrating determination of success or failure of the registration process according to a third embodiment;
[Figure 12]

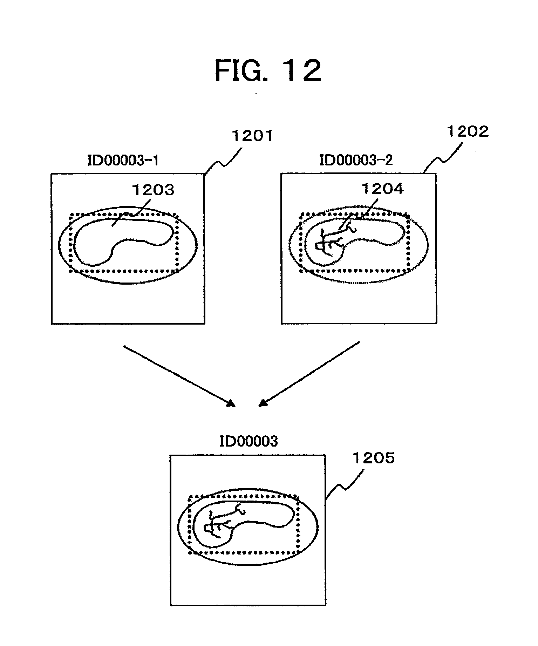

Figure 12 is a schematic diagram illustrating a modified embodiment managing a model image including two different images as a collective model image.

[Figure 13]

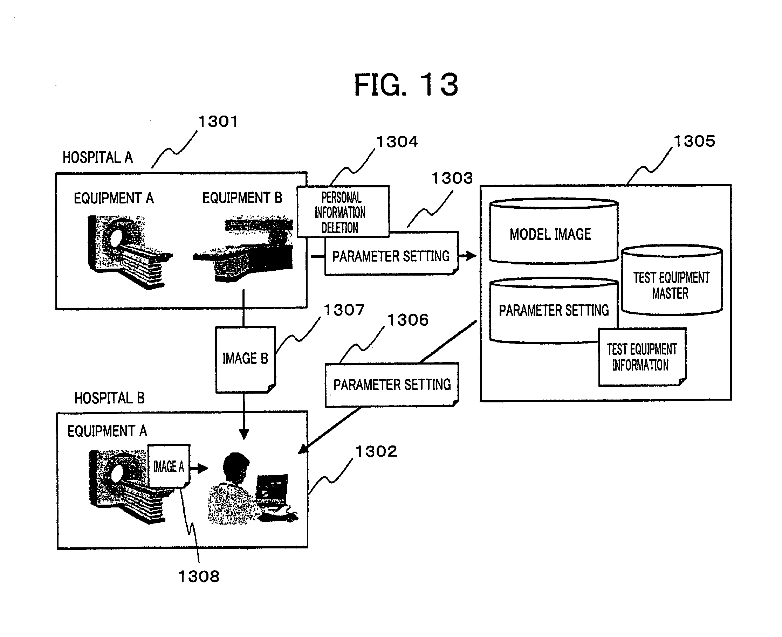

Figure 13 is a diagram schematically illustrating one configuration of the image diagnosis assisting system according to a fourth embodiment;
[Figure 14]

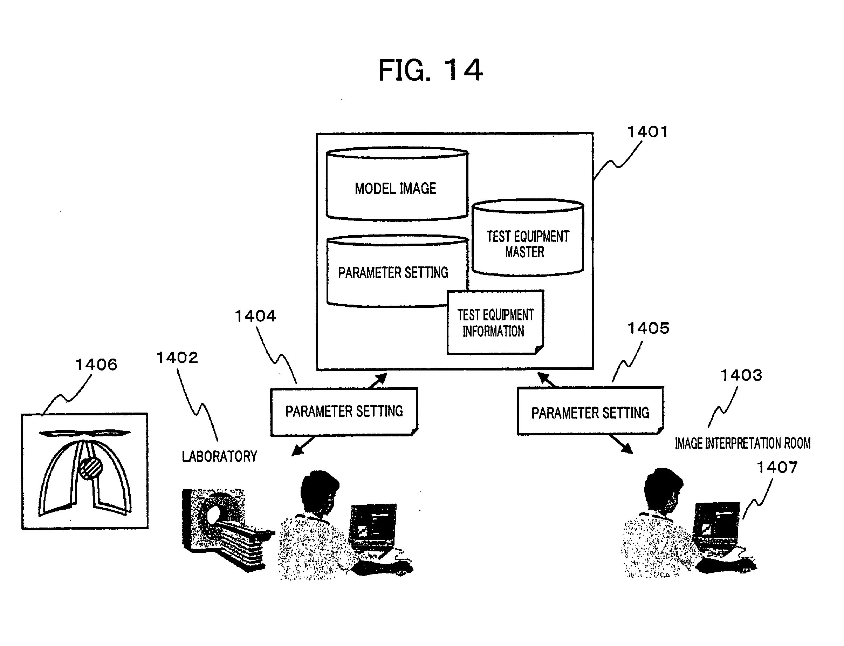

Figure 14 is a diagram schematically illustrating one configuration of the image diagnosis assisting system according to a fifth embodiment;
[Figure 15]

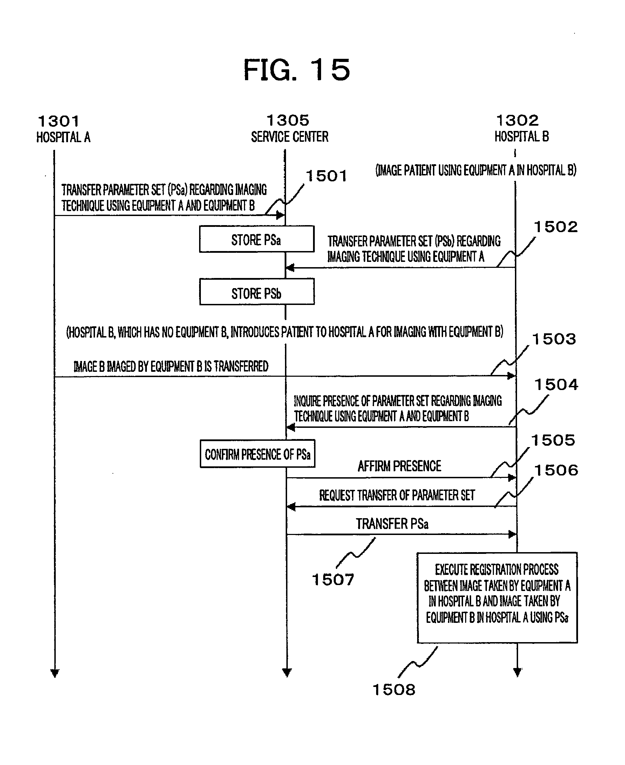

Figure 15 is a diagram showing an example of the registration process procedure using a service center according to the fourth embodiment;
[Figure 16]

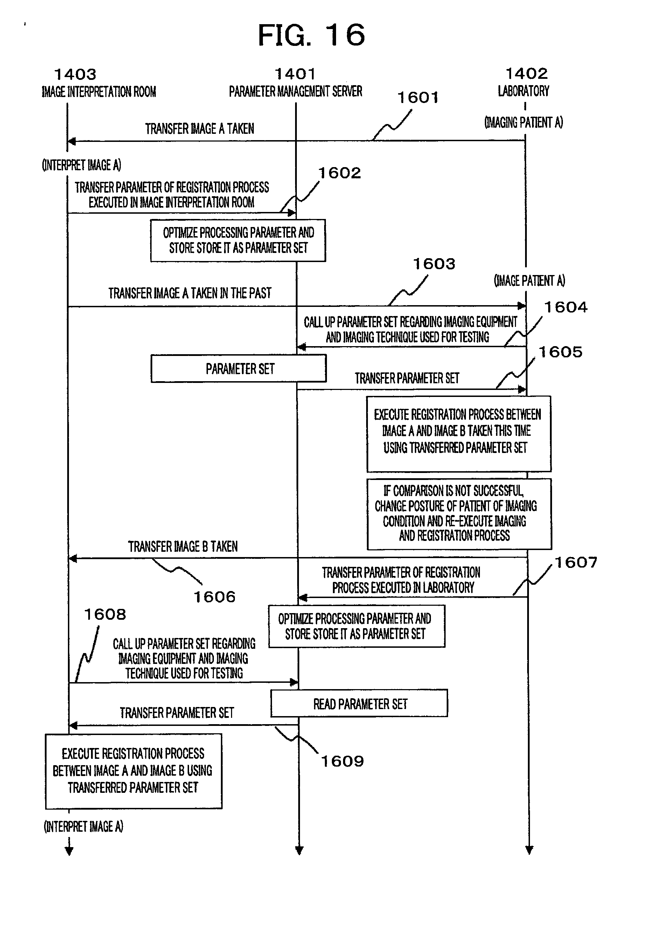

Figure 16 is a diagram showing an example of the registration process at the time point of testing according to the fifth embodiment; and
[Figure 17]

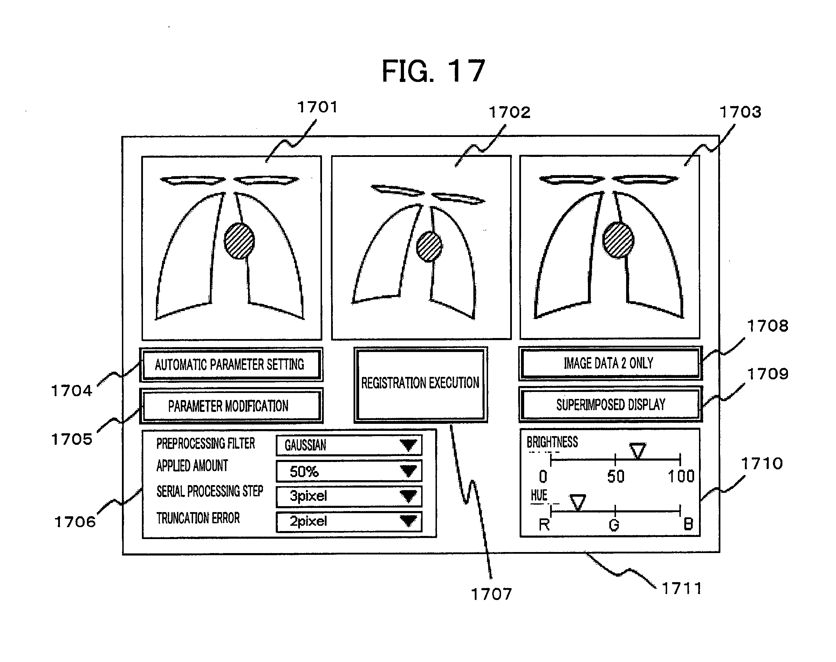

Figure 17 is a diagram showing an example of a registration process screen on a display unit according to each embodiment.

DESCRIPTION OF EMBODIMENTS

Hereinafter, various embodiments of an image diagnosis assisting system and an image diagnosis assisting apparatus to implement the present invention will be described with reference to drawings. As used herein, the image diagnosis assisting system means a system including the image diagnosis assisting apparatus and a test equipment (modality) connected to the apparatus via a network for various image diagnoses. On the other hand, the image diagnosis assisting apparatus means the apparatus excluding the test equipment, but it can include a storage device that stores therein images taken by various test equipments as well as various data. Moreover, a registration process means a process of executing an alignment between a plurality of images by moving, scaling, rotating, or deforming one of the plurality of images. When executing the registration process using the model image, a parameter set (PS) is used as various data, which may be referred to simply as a parameter. Furthermore, the test equipment and an imaging technique used to take images for various image diagnoses may be collectively referred to as a test technique.

First Embodiment

A first embodiment relates to an image diagnosis assisting system that sets a parameter for executing a registration process using a model image. Namely, the embodiment relates to an image diagnosis assisting apparatus that assists an image diagnosis by a registration process between a plurality of images, including:

a processing unit executing a registration process; and
a storage unit storing therein a parameter used for the registration process corresponding to a test technique, wherein the processing unit executes the registration process between the plurality of images using the parameter of the registration process selected based on the test technique for the plurality of images. This embodiment also relates to an image diagnosis assisting apparatus that assists an image diagnosis by a registration process between a plurality of images and a method of operating the same, which apparatus includes a processing unit executing a registration process and a storage unit storing therein a model image used for the registration process, wherein the processing unit is configured to execute the registration process between the plurality of images using an alignment reference area set to the model image selected based on the test purpose and the target site of the plurality of images and a parameter of the registration process determined based on the test technique for the plurality of images.

Figure 1A is a configuration diagram showing an example of the image diagnosis assisting system according to the first embodiment. In Figure 1A, denoted by 101 is a storage device that stores therein image data taken by a test equipment (modality) generally for various image diagnoses, and 102 is an image storage server that stores the image data in the storage device 101 and manages the image data. Similarly, denoted by 103 is a storage device that stores therein information required for implementation of the process by the image diagnosis assisting system according to the embodiment, such as the model image and the parameter, as a database. Denoted by 104 is an image interpretation terminal equipped with a display unit. The storage device 101 and the storage device 103 can be configured as storage units in the image storage server 102 and the image interpretation terminal 104, respectively.
Denoted by 106, 107, 108 are test equipments (modalities) for the image diagnosis such as a first CT (Computed Tomography) device, a second CT device, and an MRI (Magnetic Resonance Imaging) device, respectively. All of these devices are connected to one another via a network 105. The image storage server 102 and the image interpretation terminal 104 are both standard computers which include a central processing unit (CPU), a storage unit, an input/output unit such as a display unit and a keyboard, a network interface, and the like inside it.

Figure 1B shows a specific example of the image interpretation terminal 104 in the image diagnosis assisting system shown in Figure 1A, where 111 denotes a main memory (MM) acting as the storage unit, 112 denotes the CPU, 113 denotes a liquid crystal display (LCD) acting as the display unit, 114 denotes a hard disk drive (HDD), 115 denotes an input unit (INPUT) such as the keyboard, and 116 denotes the network interface (I/F). As described above, the HDD 114 can also be used as the storage device 103 and further as the storage device 101 in the system shown in Figure 1A. Although detailed explanation is omitted here because the image storage server 102 has the similar configuration, the internal HDD may be used as the storage device 101 in the system shown in Figure 1A as described above.

With the image diagnosis assisting system according to the embodiment, at first, a parameter of the registration process is set independently according to factors of the test purpose, the test technique such as the test equipment (modality) and imaging technique, and the target site, using the input unit 115 of the image interpretation terminal 104.

Hereinafter, a specific example of the registration process with the image diagnosis assisting system according to the embodiment will be described with reference to Figures 2 and 3.

Figure 2 is a flow chart showing a processing procedure of the registration process by the image interpretation terminal 104 and the like of the system according to the embodiment. Figure 3 is a schematic diagram illustrating the registration process in the system according to the embodiment, in which 301 denotes a processing unit that executes the registration process. The processing unit 301 corresponds to the CPU 112 of the interpretation terminal 104 shown in Figure 1B. First, as shown in the flow chart of Figure 2, when the registration process 201 starts, the processing unit 301 reads the test technique representing each factors described above, namely the test equipment, an imaging method 302, a test purpose 303, and a target site 304 from the database in the storage device 103 based on a user instruction input from the input unit 115 of the image interpretation terminal 104 (202), and stores them in the storage unit in the image interpretation terminal 104. Similarly, the image interpretation terminal 104 reads image data 1 and image data 2 from the test equipment stored in the storage device 101 (203, 204) and stores them in the internal storage unit. In this embodiment, the image data 1 is data of an image to be superimposed, and the image data 2 is data of an image to superimpose.

Subsequently, the processing unit 301 of the image interpretation terminal 104 determines a model image candidate from the test purpose and the target site input previously (205). The data of the model image is, as illustrated in Figure 4 later, associated with the test purpose and the target site and stored in the storage device 103. In a case where a diagnostic target is a human, as illustrated in Figure 3, the target site 304 may include, a head, a chest, a back, an abdomen, an upper limb, a lower limb and the like, as well as organs such as a cerebral cortex, a brain stem, a lung, a heart, a liver, a kidney, and the like. Next, a parameter setting unit 3013 of the processing unit 301 determines a parameter set (PS) from the test technique of the image data 1 and each model image candidate (206). This determination technique for the parameter set (PS) will be explained later with reference to Figure 5.

Then using the determined parameter set (PS), the registration process of aligning each model image with the image data 1 by moving, scaling, rotating, or deforming it with respect to the whole image is executed between the image data 1 and the model image candidates (207), thereby determining whether there is a model image successful in the registration process (208). Here, as described above, the registration process between the image data 1 and the model image candidates can proceed by sequentially reading the data of the model image candidates from the storage device 103 based on a model image ID 404. The technique for determining whether the registration process is successful will be described later.

Subsequently using the model image successful in the registration process, an alignment reference area is set. When there are a plurality of successful model images, a model image in which the shape of the site matches better is selected, which is the model image having the largest mutual information amount (209). That is, one that has the largest mutual information amount is selected from among a plurality of model image candidates including deformation or loss of the site. Then, the site on the image data 1 corresponding to the alignment reference area of the target site preset to the selected model image is set as the alignment reference area for the registration process between the image data 1 and the image data 2 (210).

The parameter for the registration process is determined from the test technique, i.e. the test equipment and the imaging method, of the image data 1 to be superimposed and the image data 2 to superimpose (211). The registration process between the image data 1 and the image data 2 is executed using the determined parameter (212) and the registration image 307 is obtained to terminate the process (213).

The determination of the parameter and the registration process at Steps 211, 212 are executed in the same manner as the procedure similar to the registration process with the model image candidate described above. Specifically, the registration is executed between the image data 1 and the image data 2 assuming that the model image candidate is the image data 2. However, although the registration process was executed with respect to the whole image at Step 207, the registration should be executed so that the alignment reference area is displayed at the same position. It should also be noted that the parameter set is selected from the test technique of the image data 1 and the image data 2. In the registration process, the rotation and/or the scaling of the image data 2 should be determined, for example, so that the mutual information amount is the maximum.

The registration process at Step 212 is now described in detail. There are generally two types of registration process: a rigid body registration to execute an alignment by moving, scaling, and/or rotating one image assuming that a shape of an object will not change; and a non-rigid body registration to execute the deformation process on the image as well assuming that the shape of the object may change. To execute the rigid body registration, the image data 2 is moved, scaled, and/or rotated so that at least the alignment reference area is displayed at the same position.

To execute the non-rigid body registration accompanying the deformation process on the image, a portion of the image data 2 corresponding to the alignment reference area set to the image data 1 is moved, scaled, and/or rotated so that at least the alignment reference area is displayed at the same position. In this case, in the image data 2, a distinct border must be generated between the inside and outside of the reference area. To relieve the border, the mutual information weighted depending on the position of pixels from the inside toward the outside of the reference area is used.

As described above, because not the whole image but only the alignment reference area can be aligned, such a problem that the alignment of the reference area cannot be executed precisely or that an error increases can be eliminated by including other organs not subjected to the diagnosis imaged around the reference area in the alignment, thereby presenting a remarkable effect that the alignment between images can be executed in a shorter time, more precisely, and more easily.

In the above process flow, when there is no model image successful in the registration process at Step 208, a new alignment reference area is set on the image data 1 to be superimposed (214), the image data 1 is added to the model image candidates (215), and the remaining steps are executed from Step 205. In other words, in this embodiment, if there is not a suitable model image, the registration process is executed by adding a new model image using image data already taken.

An example of a model image candidate table 401 used for the image diagnosis assisting system according to the embodiment is shown in Figure 4. The model image candidate table 401 is stored in the storage device 103, allowing for selection of a model image by selecting a candidate for the model image from the test purpose 402 and the target site 403. The test purpose 402 can also include a post-operative evaluation, a follow-up, a discriminable diagnosis, and the like. The post-operative evaluation may employ the same or different modality and imaging technique. The follow-up basically employs the same modality and imaging technique in most cases. For the differential diagnosis, one or both of the modality and the imaging technique is/are basically different in most cases.

In this figure, a column of the target site 403 includes the head, the lung, and the liver. A column of the model image ID 404 stores therein an identifier (ID) corresponding to each model image. The test purpose 402 and the target site 403 are assigned with the corresponding model image ID 00003-1 and 00003-2, which is an example of managing the model image including a plurality of images as a collective model image and this will be explained later with reference to Figure 12.

Figure 5 shows an example of a parameter set (PS) setting table 501 used by the image diagnosis assisting system according to the embodiment. The PS setting table 501 is stored in the storage device 103, and it is a table for determining the parameter set by the test technique, i.e. the test equipment and the imaging technique, indicating the test technique of the corresponding image in both rows and columns. The PS setting table 501 indicates simple CT, contrast enhanced CT, MR (T1), MR (T2), MR (contrast enhanced), and MR (MRA) as the test techniques, allowing for setting of the corresponding parameter set (PS) by the combination of the row and the column. In the case of Step 206, the parameter set (PS) can be determined by combining the respective test techniques for the image data 1 and the model image.

The parameters for the system according to the embodiment may include set values such as, for example, a sampling size, as well as a filter type such as a Gaussian filter that smooths an image, a coefficient, an applied amount, a number of times executing a rough registration executed as a preprocessing, a resolution of a histogram for calculating the mutual information amount, a moving width of the image in a serial processing to execute an alignment, and a truncation error.

By preparing the parameter set depending on the combination of the test techniques for the image to be registered, an appropriate parameter setting can be executed with respect to each combination of the test techniques in the registration process, which presents the remarkable effect that the alignment between images can be executed in a shorter time, more precisely, and more easily.

As shown in Figure 3, the processing unit 301 of the image diagnosis assisting system according to the embodiment determines a model image 3011, and modifies the parameter (3012) after executing the registration (3014) after the parameter setting 3013. At the parameter modification 3012, the setting of the parameter is modified according to an altered item being an execution result of the registration process in the past and the mutual information amount. Furthermore, the maximum value is set so that the modification may not be excessive. Otherwise, an amount to be reflected may be gradually increased or decreased.

Figure 6 shows an example of an execution result accumulation table 601 that accumulates therein the execution result of the registration process in the past stored in the storage device 103. The execution result accumulation table is prepared for each parameter set corresponding to each combination of the test techniques shown in Figure 5. In this figure, denoted by 602, 603, 604 are a model image ID, a registered date and time, and a number of applications, respectively. Furthermore, denoted by 605, 606, 607 are the process ID for the number of applications 10, the parameters thereof, and the mutual information amount, respectively, corresponding to the model image ID ID00001. Moreover, sets of an initial value and an applied value after the modification for a sampling interval and a Gaussian filter applied amount are respectively accumulated as the parameters. The mutual information amount 607 is normalized with 0 to 1, with the value increasing as the shape of the site matches better.

Figure 7 is a diagram illustrating an example of the parameter modification 3012 shown in Figure 3 according to the embodiment. In the parameter modification graph 701 in this figure, a horizontal axis indicates a normalized mutual information amount, and a vertical axis indicates a coefficient X. As shown in the graph 701, by providing the maximum value or gradually increasing or decreasing the reflected amount, the modification can be prevented from being excessive. In this specific example, the new initial value is determined by Equation 1 below.

```
New initial value = current initial value + (current

initial value - applied value) * coefficient X      (Equation 1)
```

The coefficient X in the above equation is the value on the vertical axis in Figure 7. By applying this equation, as an example of which is shown in the execution result accumulation table 601 in Figure 6, the parameter modification (3012) can be executed so that, for example, the modification is not reflected on the Gaussian applied amount (initial value) between a process ID 4 and a process ID 5 when the mutual information amount is small, and that the modification is reflected as between the process ID 5 and a process ID 6 when the mutual information amount is large.

In this manner, because the maximum value can be provided to the parameter modification or the modification is possible with the reflected amount gradually increased or decreased, not only an influence by the parameter modification executed immediately before but also an appropriate modification can be applied gradually, thereby presenting the remarkable effect that the alignment between images can be executed in a shorter time, more precisely, and more easily. Moreover, because it is made possible to adjust the modified amount of the parameter using the mutual information amount, not only the value of the parameter setting in the past but also the set value of the parameter when the images are aligned better can be reflected on the modification, which allows for application of more appropriate parameter modification, thereby presenting the remarkable effect that the alignment between images can be executed in a shorter time, more precisely, and more easily.

Furthermore, because such a modification is executed on each parameter set corresponding to the test technique, the appropriate parameter modification can be executed with respect to each combination of the test technique without being influenced by the parameter modification executed using another combination of the test technique, thereby presenting the remarkable effect that the alignment between images can be executed in a shorter time, more precisely, and more easily.

Moreover, when the sampling interval is updated as between a process ID 8 and a process ID 9, all the initial values can be changed to the last set values. Because the change of the sampling interval is not in a linear relation with another processing parameter in many cases, such a different type of change is executed instead of the modification by gradually increasing or decreasing the value as described above. Thus, an appropriate modification can be applied to each type of the parameters, thereby presenting the remarkable effect that the alignment between images can be executed in a shorter time, more precisely, and more easily.

Figures 8A and 8B show examples of the model image in this embodiment. The processing unit 301 of the image diagnosis assisting system described above needs to register and manage the model image according to the test purpose and the target site in the registration process that is the alignment between images. The model images shown in Figures 8A and 8B show model images 801, 803 of the whole lung field of a human subject. Thus, when the alignment process is executed on the whole lung field, the registration process of the image of which target site is the lung is likely to be affected by respiration, and therefore matching the position of an organ of the upper portion which is less affected by the respiration can improve an accuracy of the registration process that is the alignment between images.

Now in Figure 8A, the position of the organ of the upper portion with less body motion by the respiration, such as a rib, is regarded as an alignment reference area 802, and the model image 801 is matched with the alignment reference area 802. As shown in Figure 8B, to diagnose a disease of a bronchus in the model image 803, the diverging points of the bronchus are regarded as alignment reference areas 804-1 to 804-5, and the images are matched so that as many of these alignment reference areas can match, thereby improving the accuracy of the registration process that is the alignment between images.

It should be noted that the images may not always be correctly aligned in areas other than the alignment reference areas. However, it may not be a significant problem depending on the test purpose. For example, when interpreting the whole lung field as described above, there may be a mismatch between images due to an influence by the respiration or a heartbeat in the areas other than the reference areas, but these motions are inherent to a human body which are often taken into account for interpreting the images, resulting in only an insignificant problem depending on the test purpose.

As another example, the motion itself can be an object of the image interpretation. For example, when diagnosing a function of skeletal muscles, a fulcrum of a joint connecting the skeletal muscles is set as an alignment reference area and the skeletal muscles are set as the areas other than the alignment reference area to align the image data taken at the times of contraction and relaxation of the muscles based on the joint (that is not the skeletal muscle), which allows for more correct diagnosis of the function of the skeletal muscles. Thus in the embodiment, because the alignment reference area (e.g., rib or joint) different from the target site can be set according to the test purpose and the target site, there is a remarkable effect that an exact alignment can be executed even when executing the image diagnosis as described above.

Because the model image can be registered and managed according to the test purpose and the target site in this manner, an appropriate model image can be selected according to the test purpose and the target site such as, for example, the whole lung field when interpreting the image focusing on the whole lung field for lung cancer or the diverging point of the bronchus when interpreting the image focusing on the bronchi for bronchitis, which can improve the accuracy of the registration process for determining the target site and further presents a remarkable effect that the alignment between images focusing on the target site can be executed in a shorter time, more precisely, and more easily.

Furthermore, model images having different alignment reference areas can be registered and managed according to the test purpose and the target site. Thus, because an alignment area different from the target site can be set according to the test purpose, it is possible to execute the registration process more useful for the diagnosis by, for example, setting the organ such as the rib in the upper portion less influenced by the body motion due to the respiration in the case of the whole lung field, thereby presenting the remarkable effect that the alignment between images focusing on the target site can be executed in a shorter time, more precisely, and more easily.

Figure 9 shows a state in which the data of the model image shown in Figures 8A and 8B is stored in the storage device 103 of the image diagnosis assisting system in Figure 1. In the case of this embodiment, the alignment reference area in the model image is represented by area data of an area surrounded by a rectangle. In Figure 9, the target site in the model image candidate table 401 is the lung, and the model images ID00002 and ID00025 respectively indicate the data of the alignment reference areas 802, 804 of the model images in Figures 8A and 8B. The storage device 103 stores therein area data 901, 902 corresponding to the alignment reference areas 802, 804 of these model images. The area data 901, 902 are respectively constituted by area ID, type ID, origin, and size. Here, the area ID means an ID for identifying the alignment reference area in the model image. In the column of type ID, "0" means an added area and "1" means a deleted area. Origin means a point of origin of the reference area, and size means the size of the reference area. In the case of the area data 902 shown in Figure 8B, the area IDs corresponding to the alignment reference areas 804-1 to 804-5 are 1 to 5. In Figure 9, the area data 903 is the data associated with a second embodiment, which will be described later.

Figure 10 is a diagram illustrating an example of the alignment reference area of the target site and the three-dimensional data management method for the model image. Although the explanations of Figures 8A to 8E and Figure 9 were given using the two-dimensional image data showing the model images on a predetermined plane in a three-dimensional coordinate system for simplifying the illustration, image data obtained from a test equipment such as the CT device and the MRI device is essentially three-dimensional data in many cases. Therefore, Figure 10 schematically shows a rectangular reference area 1002 in an area 1001 of a taken image formed with reference to the origin (0, 0, 0) of the XYZ three-dimensional coordinate system of the image. The rectangular reference area 1002 has an origin $(x0, y0, z0)$ and a size $(x1, y1, z1)$.

Because combining a plurality of two-dimensional or three-dimensional rectangular areas thus makes it possible to set the alignment reference area of the target site, it is now possible to set an alignment reference area in a single area and a complicated alignment reference area straddling a plurality of areas like the diverging point of the bronchus, thereby presenting the remarkable effect that the alignment between images can be executed in a shorter time, more precisely, and more easily even when employing different test purpose or target site.

As described above, in this embodiment, because the parameter set is prepared depending on the combination of the test technique for the image to be registered, an appropriate parameter setting can be executed with respect to each combination of the test technique in the registration process, thereby presenting the remarkable effect that the alignment between images can be executed in a shorter time, more precisely, and more easily.

Furthermore, because the model image can be registered and managed according to the test purpose and the target site, an appropriate model image can be selected depending on the test purpose and the target site, thereby presenting the remarkable effect that the alignment between images focusing on the target site can be executed in a shorter time, more precisely, and more easily.

Moreover, because not only an influence by the parameter modification executed immediately before but also an appropriate modification can be applied gradually, there is the remarkable effect that the alignment between images can be executed in a shorter time, more precisely, and more easily. Furthermore, because it is made possible to adjust the modified amount of the parameter using the mutual information amount, the set value of the parameter when the images are aligned better can be reflected on the modification, which allows for application of more appropriate parameter modification, thereby presenting the remarkable effect that the alignment between images can be executed in a shorter time, more precisely, and more easily.

When the registration process is executed between the image data 1 and each model image candidate at Step 207 in this embodiment, the model image is fit in the image data 1 by moving, scaling, rotating, or deforming the model image with respect to the whole image, but it may be fit in another way. For example, when only a specific site which can be less deformed such as the head is targeted, the fitting may be executed using the rigid body registration process without deformation, or the registration process targeting only an alignment reference area preset to the candidate for the model image may be executed instead of executing the registration process with respect to the whole image. It is possible to optimize the processing procedure according to the feature of the target image data or the model image candidate.

Second Embodiment

Subsequently, a second embodiment is described. The second embodiment relates to an image diagnosis assisting system that automatically sets the alignment reference area of the target site by automatically selecting the optimal one from a plurality of model images including deformation or loss of the site. That is, the embodiment relates to an image diagnosis assisting apparatus that assists an image diagnosis by a registration process between a plurality of images, the image diagnosis assisting apparatus including a processing unit executing a registration process and a storage unit storing therein a model image used for the registration process, wherein the processing unit is configured to set an alignment reference area by automatically selecting it from among a plurality of model images including deformation or loss of a site in the image, and to execute the registration process between the plurality of images using the set alignment reference area and a parameter of the registration process determined based on the test technique for the plurality of images.

Figures 8C, 8D, and 8E show the model images as in Figure 8A, while Figures 8C and 8D show examples in which new model images 805, 808 are added. They are examples of adding a new model image by modifying the setting of the current alignment reference area when the database in the storage device 103 or the like does not include an appropriate model image. Figure 8E shows an example in which a user can interactively set an image of a target area when generating a new model image 810.

In Figure 8C, when a heart 806 is large and an area of an alignment reference area 802 is too large in a model image 801 on the database to separate the heart 806, by setting a still upper area as an alignment reference area 807, it is possible to add a model image 805 with reduced influence by an individual difference. In Figure 8D, if there was a disease in the past and one lung has been removed, it is possible to add a model image 808 with reduced influence by the medical treatment by setting only the other lung as an alignment reference area 809.

As described above, even when there is an individual difference of the organ in shape and size, an influence from the past treatment, a congenital malformation, or the like, the alignment reference area of the target site can be set only by adding a model image, presenting the remarkable effect that the alignment between images can be executed in a shorter time, more precisely, and more easily.

Figure 8E shows a case of adding the new model image 810 with a part of the alignment reference area of the target site eliminated from the alignment reference area of the target site in an existing model image by the user interactively specifying the area desired to separate. In other words, a part of the area can be eliminated by specifying an area 812 to be separated from the alignment reference area of the existing target site to create an alignment reference area 811 of a new site.

A case of storing the data of the added model image in the storage device 103 is explained with reference to Figures 8E and 9. As shown in Figure 9, area data 903 of the model image 810 includes areas of the alignment reference area 811, as well as the type ID, the origin, and the size of area ID 1, ID 2 corresponding to the area 812 to be separated. Because the area to be separated can be thus interactively specified by combining rectangular areas, it is now possible to specify the target area difficult to specify with a single rectangular area such as a site surrounded by other organs or a site inside a complicated anatomy of human body enabling addition and utilization of various model images, thereby presenting the remarkable effect that the alignment between images can be executed in a shorter time, more precisely, and more easily.

The specification and addition of the alignment reference area of the target site described in this embodiment can be executed at Steps 214 and 215 in Figure 2 described in the first embodiment. Furthermore, the parameter initial value of the newly added model image is determined based on the value set to an image with the same test technique.

Third Embodiment

Next, as a third embodiment, an image diagnosis assisting system is described that can determine a success or failure of the registration process based on a value of or a change of the mutual information amount in a serial processing step in the alignment. In other words, the embodiment relates to the image diagnosis assisting apparatus in which the processing unit of the aforementioned image diagnosis assisting apparatus determines the success or failure of the registration process executed between a candidate for the model image and a first image based on the value of the mutual information amount obtained in the registration process. The system configuration per se is the same as the system in the embodiment described above, and therefore an explanation thereof is omitted here.

Figure 11 shows a graph for illustrating the determination of the success or failure of the registration process in this embodiment. The horizontal axis of the graph in the figure indicates the number of alignment processes in the registration process, and the vertical axis indicates the mutual information amount. A curve 1101 indicates the mutual information amount in a case where both the test technique and the target site match, and a curve 1102 indicates the mutual information amount in a case where only the target site matches. 1103 indicates a threshold of the mutual information amount for determining the success or failure of the alignment. Both curves 1101 and 1102 indicate a tendency of increasing the mutual information amount as the number of alignment processes increases.

When the mutual information amount exceeds the preset threshold for determining the success or failure by increasing the number of alignment processes, the processing unit 301 in the image interpretation terminal 104 determines that the registration process was successful. When the mutual information amount does not change at all even if the number of alignment processes increases, the processing unit 301 can recheck whether the same processing result is obtained by forcing one image to move. In the case of the curve 1101 where the test technique and the target site match between images, the threshold of the mutual information amount for determination of the success or failure is set higher (1104).

As described above, in this embodiment, because the mutual information amount for determining the success or failure of the alignment can be changed depending on the match or unmatch of the test technique of the superimposed images, the determination of the success or failure can be executed more correctly, presenting the remarkable effect that the alignment between images can be executed in a shorter time, more precisely, and more easily.

In this embodiment, the determination of the success or failure can be executed in combination with another index. For example, using information of change of the mutual information amount with respect to the number of processes, a condition may be added that the process is successful when the change is no higher than a preset threshold. This presents an effect of improving the accuracy of the determination of the success or failure.

In each embodiment described above, the explanation was given assuming that the model image is basically formed corresponding to each test equipment and imaging technique, but the model image can be managed by organizing images of a plurality of imaging techniques together. This variation is explained with reference to Figure 12.

Figure 12 is a schematic diagram showing an example of managing model images including two different images as a collective model image, as a variation. There may be a case of taking a plurality of images using different imaging techniques, for example a CT image and a contrast enhanced CT image, in a single test such as a liver test, and making a diagnosis based on the result thereof. In this case, a model image 1201 as the CT image and a model image 1202 as the contrast enhanced CT image are collectively managed. Here they are managed as a CT model image ID 00003-1 and a contrast enhanced CT image ID 00003-2, respectively. Denoted by 1203 is the liver and 1204 is a portal vein. By managing the images using the plurality of imaging techniques collectively, the registration between images using the same imaging technique is more likely to be aligned more precisely, which can improve the accuracy of the alignment compared with a case of executing the registration individually, thereby presenting the remarkable effect that the alignment between images can be executed in a shorter time, more precisely, and more easily.

Furthermore, as another variation, it is also possible to generate a new model image 1205 of a model image ID 00003 by superimposing both images of the model image 1201 and the model image 1202 and make use of it as the model image. In this case, the same model image can be applied to a plurality of images using different imaging techniques, which can reduce the cost of management of the model image or the registration process, thereby presenting the remarkable effect that the alignment between images can be executed in a shorter time, more precisely, and more easily. In this case, it is preferable to prepare a dedicated parameter set (PS) assuming an image by a new test equipment (modality).

Fourth Embodiment

Subsequently, as a fourth embodiment, an image diagnosis assisting system capable of executing an optimization of the parameter set (PS) for the registration process used in the aforementioned embodiments in a plurality of hospitals is described. This embodiment collects and manages the setting status of the processing parameter in the plurality of hospitals and constructs the database (DB) including appropriate parameters according to the imaging technique in a service center.

Figure 13 is a diagram schematically illustrating a system configuration according to the fourth embodiment. In a plurality of hospitals A 1301 and B 1302, a test equipment A and a test equipment B for executing an image diagnosis operate and obtain an image A1308 and B1307, respectively. The plurality of hospitals A 1301 and B 1302 are connected to a service center 1305 via an unshown network or the like through which various data can be transferred. The service center 1305 includes an unshown server having a standard computer configuration, i.e., including a processing unit, a storage unit, an input/output unit, a network interface unit and the like connected to one another, wherein the storage unit stores therein a model image, data for parameter setting, various test equipment information, and test equipment master information as shown in Figure 13.

Figure 15 is a timing chart showing an example of the registration process procedure of an image taken by a system using the service center shown in Figure 13 in the hospital A 1301 and the hospital B 1302. First, the hospital A 1301 transfers a parameter set (PSa) related to the imaging technique that uses an equipment A and an equipment B to the service center 1305 (1501). The service center 1305 stores the received PSa in the storage unit of the server in the center.

Next, when the hospital B 1302 transfers a parameter set (PSb) related to the imaging technique used to take an image of a patient using the equipment A in the hospital B 1302 to the service center 1305 (1502), the service center 1305 stores the received PSb in the storage unit of the server in the center. As shown in Figure 13, because the hospital B 1302 does not have the equipment B, the hospital introduces the patient to the hospital A 1301 to have the image taken by the equipment B, and the taken image B is transferred to the hospital 1302 (1503). Upon receipt of the taken image B, the hospital B inquires the service center 1305 for the presence of the parameter set related to the imaging technique using the equipment A and the equipment B (1504).

The service center 1305 confirms the presence of the PSa and replies to the hospital B that the PSA is present (1505). In response to the reply, the hospital 1302 requests the service center 1305 to transfer the parameter set (1506), and receives the PSa (1507). As a result, the hospital B can execute the registration process between the image taken by the equipment A in the hospital B and the image taken by the equipment B in the hospital A using the received PSa.

It can be assumed here, for example, to use a CT device as the equipment A and an MRI device as the equipment B. Because the penetration number and penetration rate of the MRI device are generally lower than those of the CT device, it can be assumed that the patient is introduced to a hospital having the MRI device for executing the test. This can enable, for example, the follow-up in another hospital using an image taken at a hospital where a surgery was executed.

Thus, according to the embodiment, because the collective management of the parameter set in the service center allows for applying the optimal processing parameter to an image taken in another hospital, the parameter can be optimized beyond systems in each hospital, thereby presenting the remarkable effect that the alignment between images can be executed in a shorter time, more precisely, and more easily even when using images taken in different hospitals.

Fifth Embodiment

Next, with reference to Figures 14 and 16, there is described below as a fifth embodiment an image diagnosis assisting system capable of executing the registration process at the time of the test and of feeding back to the imaging condition for the test equipment. In this embodiment, the result of the registration process is displayed on the test equipment immediately after the imaging enabling an immediate determination of whether a deformation amount in the target area of the image is large in order to feed back the result.

In Figure 14, 1401 denotes a parameter management server, 1402 and 1403 respectively denote a laboratory and an image interpretation room connected to the parameter management server 1401 via an unshown network, 1406 denotes a model image, and 1407 denotes an image interpretation terminal.

As seen in Figure 16, a laboratory technician in the laboratory 1402 transfers an image A of a patient A taken with the test equipment 1406 in the laboratory 1402 to the image interpretation room 1403 (1601). In the image interpretation room 1403, an image reading doctor interprets the image A using the image interpretation terminal 1407, and transfers a parameter setting 1405 of the registration process executed at the time to the parameter management server 1401 (1602). The parameter management server 1401 optimizes the processing parameter and stores it therein as the parameter set.

When an image of the patient A is taken again in the laboratory 1402 later, the image A taken in the past is received from the interpretation room A (1603). The parameter set related to the imaging equipment and the imaging technique used to take the image A is called from the parameter management server 1601 (1604) and the transferred parameter set is received (1605). In the laboratory 1402, the registration process between the image A and the image B taken this time is executed using the received parameter set. If the comparison is not successful in this registration process, the posture of the patient in the imaging condition may be changed and the imaging and registration process may be executed again in the laboratory 1402. The taken image B is then transferred to the image interpretation room 1403 (1606) and at the same time the parameter of the executed registration process is transferred to the parameter management server 1401 (1607). The parameter management server 1401 optimizes the transferred processing parameter and stores it in the storage unit as the parameter set.

The image reading doctor in the image interpretation room 1403 can call the parameter set related to the imaging equipment and the imaging technique used in the test from the parameter management server 1401 through the image interpretation terminal 1407, and execute the registration process between the image A and the image B using the transferred parameter set, thereby interpreting the image B.

According to the embodiment as described above, because the parameter management server 1401 shares all the processing parameters in the hospital enabling the registration process in the laboratory making use of various cases in the hospital and also enabling more appropriate parameter set to be used immediately, there is the remarkable effect that the alignment between images can be executed in a shorter time, more precisely, and more easily. Furthermore, it is possible that the image can be retaken immediately when the deformation amount of the compared image is large, or the deformation amount can be reflected to the setting of the imaging condition of the test equipment (modality) or to the retake, thereby presenting a remarkable effect of improving the total efficiency of the image diagnosis from the image taking to the interpretation and preventing a case of requiring the retake at a later date to reduce the burden on the patient in advance.

Moreover, according to the embodiment, in a case of a patient who is regularly followed up, because the same test has been executed several times in the past using the same test equipment and the same imaging technique, the registration process may be executed every time with the image taken in the past presenting a remarkable effect of efficiently executing the image interpretation by comparing the images. Furthermore, there is a remarkable effect that the posture of the patient or the imaging condition can be optimized on the scene by executing the registration process in the laboratory 1402. Moreover, the registration process can be executed at the time of the image interpretation in the image interpretation room 1403 using the parameter setting in the registration process executed at the time of image taking enabling more appropriate parameter set to be used immediately, thereby presenting the remarkable effect that the alignment between images can be executed in a shorter time, more precisely, and more easily.

Next, an example of a registration process screen on the image diagnosis assisting system according to each embodiment described above is described below.

Figure 17 is a diagram showing an example of the registration process screen according to each embodiment described above. In the figure, a screen 1711 displays image data 1 1701, image data 2 1702, and a registration result image 1703. Arranged below these images is an automatic parameter setting button 1704, with which button the automatic setting of the parameter according to each embodiment described above can start. A parameter modification button 1705 is selected when a failure occurs during the automatic setting. A pull-down menu 1706 is a parameter setting unit which can change the preprocessing filter, the applied amount, the serial processing step, the truncation error, or the like when the parameter modification is selected. A registration execution button 1707 is a button for executing the registration process of fitting the image data 2 1702 in the image data 1 1701.

An image data 2 only button 1708 is a button for displaying only the processing result of the image data 2 on the registration result image. A superimposed display button 1709 is a button for displaying the image data 1 and the image data after the registration process superimposed on the same screen. A brightness/hue slider 1710 is a slider for altering the brightness and the hue of the image data 2 in the state of the superimposed display.

Because it is thus facilitated to visually check whether the superposition of the images has been executed well only by the button operation and the slider movement, there is the remarkable effect that the alignment between images can be executed in a shorter time, more precisely, and more easily.

It should be noted that the present invention is not limited to the embodiments described above but also encompasses various variations. For example, the above embodiments are intended to explain the invention in detail for comprehensible illustration but not to limit the invention to necessarily include all the configurations. Furthermore, a part of a configuration of one embodiment can be replaced by a configuration of another embodiment, or a configuration of one embodiment can be added to a configuration of another embodiment. Moreover, a part of a configuration in each embodiment can be added with another configuration, deleted, or replaced by another configuration.

Furthermore, a part or all of each configuration, function, processing unit, processing means, or the like described above may be implemented as hardware by, for example, designing it as an integrated circuit. Each configuration, function, or the like may also be implemented as software as described above by the process translating and executing a program that implements each function thereof. Information such as a program, a table, or a file to implement each function can be stored in a storage device such as a memory, a hard disk, or an SSD (Solid State Drive) or a recording medium such as an IC card and a DVD (Digital Versatile Disc), or it can also be downloaded via a network or the like as needed.

INDUSTRIAL APPLICABILITY

The present invention is extremely useful as an image diagnosis assisting apparatus, and specifically as a technology to improve efficiency of an alignment process between images when interpreting a plurality of images by comparison.

EXPLANATIONS OF LETTERS OR NUMERALS

| | |
|---|---|
| 101, 103 | Storage device |
| 102 | Image storage server |
| 104 | Image interpretation terminal |
| 105 | Internal bus |
| 106, 107 | CT device |
| 108 | MRI device |
| 111 | Main memory (MM) |
| 112 | Central processing unit (CPU) |
| 113 | Liquid crystal display (LCD) |
| 114 | Hard disk drive (HDD) |
| 115 | Input unit (INPUT) |
| 116 | Network interface (I/F) |
| 301 | Processing unit |
| 3011 | Model image |
| 3012 | Parameter modification |
| 3013 | Parameter setting |
| 3014 | Registration execution |
| 302 | Test equipment, imaging technique |
| 303 | Test purpose |
| 304, 403 | Target site |
| 305 | Image data 1 |
| 306 | Image data 2 |
| 307 | Registration image |
| 401 | Model image candidate table |
| 501 | Parameter set (PS) setting table |
| 601 | Execution result accumulation table |
| 701 | Parameter modification graph |
| 801, 803, 805, 808, 810 | Model image |
| 802, 804, 807, 809, 811 | Alignment reference area |
| 806 | Heart |
| 812 | Area to be separated |
| 901, 902, 903 | Area data |
| 1001 | Area of taken image |
| 1002 | Rectangular reference area |
| 1101, 1102 | Mutual information |
| 1103, 1104 | Threshold of mutual information amount |
| 1201 | Model image as CT image |
| 1202 | Model image as contrast enhanced CT image |
| 1203 | Liver |
| 1204 | Portal vein |
| 1205 | New model image |
| 1301 | Hospital A |
| 1302 | Hospital B |
| 1303, 1306 | Parameter setting |
| 1304 | Personal information deletion |
| 1305 | service center |
| 1307 | Image B |
| 1308 | Image A |

| 1401 | Parameter management server |
|---|---|
| 1402 | Laboratory |
| 1403 | Image interpretation room |
| 1404, 1405 | Parameter setting |
| 1406 | Image data |
| 1407 | Image interpretation terminal |
| 1701 | Image data 1 |
| 1702 | Image data 2 |
| 1703 | Registration result image |
| 1704 | Automatic parameter setting button |
| 1705 | Parameter modification button |
| 1706 | Pull-down menu |
| 1707 | Registration execution button |
| 1708 | Image data 2 only button |
| 1709 | Superimposed display button |
| 1710 | Hue slider |
| 1711 | Display screen |

**Claims**

1. An image diagnosis assisting apparatus that assists an image diagnosis by a registration process between a plurality of images, comprising:

   a processing unit executing the registration process; and
   a storage unit storing therein a parameter used for the registration process corresponding to a test technique, wherein
   the processing unit executes the registration process between the plurality of images using the parameter of the registration process selected based on the test technique for the plurality of images.

2. The image diagnosis assisting apparatus according to claim 1, wherein the storage unit stores therein a model image used for the registration process corresponding to a test purpose and a target site, and the processing unit selects a candidate for the model image used for the registration process based on the test purpose and the target site.

3. The image diagnosis assisting apparatus according to claim 2, wherein the processing unit determines a parameter for the registration process between the candidate for the model image and a first image based on the test techniques for the candidate for the model image and the first image among the plurality of images, and executes the registration process between the candidate for the model image and the first image.

4. The image diagnosis assisting apparatus according to claim 2, wherein the model image stored in the storage unit includes an alignment reference area for executing the registration process, and the processing unit executes the registration process in the alignment reference area of the model image selected based on the test purpose and the target site.

5. The image diagnosis assisting apparatus according to claim 3, wherein the model image stored in the storage unit includes an alignment reference area for executing the registration process, and when the registration process executed in the alignment reference area between the candidate for the model image and the first image is successful, the processing unit selects the model image from among the successful candidates for the model image and sets an area in the first image corresponding to the alignment reference area in the selected model image as an alignment reference area for executing the registration process between the first image and a second image among the plurality of images.

6. The image diagnosis assisting apparatus according to claim 5, wherein the processing unit determines the parameter for the registration process between the first image and the second image based on the test techniques for the first image and the second image.

7. The image diagnosis assisting apparatus according to claim 3, wherein, when the registration process executed between the candidate for the model image and the first image is not successful, the processing unit adds the first image to which a new alignment reference area is set to candidates for the model image.

8. The image diagnosis assisting apparatus according to claim 7, further including:

   a display unit for displaying an image, wherein
   when the registration process executed between the candidate for the model image and the first image is not successful, the processing unit displays the first image on the display unit.

9. The image diagnosis assisting apparatus according to claim 3, wherein the processing unit determines a success or failure of the registration process executed between the candidate for the model image and the first image based on a value of a mutual information amount obtained by the registration process.

10. The image diagnosis assisting apparatus according to claim 9, wherein, when the test purposes, the test techniques, and the target sites of the candidate for the model image and the first image for determining the success or failure of the registration process match, the processing unit sets a threshold of the mutual information amount higher than that in another case.

11. The image diagnosis assisting apparatus according to claim 1, wherein after executing the registration process, the processing unit modifies the parameter reflecting the execution result.

12. A method of operating an image diagnosis assisting apparatus using a terminal that assists an image diagnosis by a registration process between a plurality of images,
    wherein the terminal selects a model image based on a test purpose and a target site of the plurality of images, and the registration process between the plurality of images is executed using an alignment reference area preset to the selected model image and a parameter of the registration process determined based on a test technique for the plurality of images.

13. The method of operating the image diagnosis assisting apparatus according to claim 12,
    wherein the terminal selects a candidate for the model image used for the registration process based on the test purpose and the target site, and
    the registration process between the candidate for the model image and a first image among the plurality of images is executed in the alignment reference area.

14. The method of operating the image diagnosis assisting apparatus according to claim 13, wherein when the registration process executed in the alignment reference area between the candidate for the model image and the first image is successful, the processing unit selects the model image from among the successful candidates for the model image and sets an area in the first image corresponding to the alignment reference area in the selected model image as an alignment reference area for executing the registration process between the first image and a second image among the plurality of images.

15. The method of operating the image diagnosis assisting apparatus according to claim 12, wherein the terminal executes the registration process in the alignment reference area of the model image selected based on the test purpose and the target site.

16. The method of operating the image diagnosis assisting apparatus according to claim 13, wherein when the registration process executed between the candidate for the model image and the first image is not successful, the terminal adds the first image to which a new alignment reference area is set to candidates for the model image.

17. The method of operating the image diagnosis assisting apparatus according to claim 13, wherein the terminal determines a success or failure of the registration process executed between the candidate for the model image and the first image based on a value of a mutual information amount obtained by the registration process.

# FIG. 1A

102

IMAGE
STORAGE
SERVER

101

105

106

STORAGE DEVICE

103

FIRST CT DEVICE

107

STORAGE DEVICE

SECOND CT DEVICE

108

104

IMAGE INTERPRETATION
TERMINAL

MRI DEVICE

# FIG. 1B

111

112

113

MM

CPU

LCD

117

114

115

116

HDD

INPUT

I/F

# FIG. 2

```
REGISTRATION PROCESS                                    201

READ TEST PURPOSE, TEST EQUIPMENT,                     202
IMAGING METHOD, TARGET SITE

READ IMAGE DATA 1                                      203

READ IMAGE DATA 2                                      204

DETERMINE MODEL IMAGE CANDIDATE FROM                   205
TEST PURPOSE AND TARGET SITE

DETERMINE PARAMETER FROM TEST EQUIPMENT                206
AND IMAGING METHOD OF IMAGE DATA 1 AND
EACH MODEL IMAGE CANDIDATE

EXECUTE REGISTRATION BETWEEN IMAGE                     207
DATA 1 AND EACH MODEL IMAGE CANDIDATE

THERE IS MODEL IMAGE SUCCESSFUL      NO
IN REGISTRATION                                        208

                              YES

SELECT MODEL IMAGE WITH MAXIMUM MUTUAL                 209
INFORMATION AMOUNT IF THERE ARE
A PLURALITY OF SUCCESSFUL MODEL IMAGES

SET SITE ON IMAGE DATA 1 CORRESPONDING                 210
TO ALIGNMENT REFERENCE AREA OF TARGET
SITE SET ON MODEL IMAGE AS ALIGNMENT
REFERENCE AREA FOR REGISTRATION

DETERMINE PARAMETER FROM TEST                          211
EQUIPMENT AND IMAGING METHOD OF
IMAGE DATA 1 AND IMAGE DATA 2

EXECUTE REGISTRATION BETWEEN IMAGE DATA 1              212
AND IMAGE DATA 2 (DETERMINE ROTATION AND
MAGNIFICATION OF IMAGE DATA 2 ALLOWING
MAXIMUM MUTUAL INFORMATION AMOUNT)

END                                                    213

                                              214
SET ALIGNMENT REFERENCE AREA OF
TARGET SITE ANEW ON IMAGE DATA 1

ADD IMAGE DATA 1 TO
MODEL IMAGE CANDIDATES
                                              215
```

# FIG. 3

TEST EQUIPMENT, IMAGING TECHNIQUE — 302

TEST PURPOSE — 303

TARGET SITE
(1) SITE SUCH AS HEAD, CHEST, BACK,
    ABDOMEN, UPPER LIMB, LOWER LIMB, ETC.
(2) ORGAN SUCH AS CEREBRAL CORTEX,
    BRAIN STEM, LUNG, HEART, LIVER, KIDNEY, ETC. — 304

IMAGE DATA 1 — 305

IMAGE DATA 2 — 306

PROCESSING UNIT — 301

3011 — MODEL IMAGE

PARAMETER MODIFICATION — 3012

PARAMETER SETTING UNIT — 3013

REGISTRATION EXECUTION UNIT — 3014

REGISTRATION IMAGE
(RESULT OF PROCESSING IMAGE 2) — 307

EP 2 719 325 A1

# FIG. 4

| TEST PURPOSE | TARGET SITE | MODEL IMAGE ID |
|---|---|---|
| FOLLOW-UP | HEAD | ID00001 |
| | | ID00002 |
| | | ID00005 |
| | | ID00023 |
| | | ID00041 |
| | LUNG | ID00002 |
| | | ID00025 |
| | | ID00044 |
| DIFFERENTIAL DIAGNOSIS | LIVER | ID00003-1 ID00003-2 |
| | | |
| | | |
| | | |

402 403 404 401

# FIG. 5

| | SIMPLE CT | CONTRAST ENHANCED CT | MR(T1) | MR(T2) | MR (CONTRAST ENHANCED) | MR (MRA) |
|---|---|---|---|---|---|---|
| SIMPLE CT | PS11 | PS12 | PS13 | PS14 | PS15 | PS16 |
| CONTRAST ENHANCED CT | PS21 | PS22 | PS23 | PS24 | PS25 | PS26 |
| MR(T1) | PS31 | PS32 | PS33 | PS34 | PS35 | PS36 |
| MR(T2) | PS41 | PS42 | PS43 | PS44 | PS45 | PS46 |
| MR (CONTRAST ENHANCED) | PS51 | PS52 | PS53 | PS54 | PS55 | PS56 |
| MR(MRA) | PS61 | PS62 | PS63 | PS64 | PS65 | PS66 |
| | | | | | | |

501

# FIG. 6

| MODEL IMAGE ID | REGISTERED DATE AND TIME | NUMBER OF APPLICATIONS |
|---|---|---|
| ID00001 | 20101201 | 10 |
| ID00002 | 20101206 | 4 |
| | | |
| | | |
| | | |

602  603  604

601

605  606  607

| PROCESS ID | PARAMETER | | | | MUTUAL INFORMATION AMOUNT |
|---|---|---|---|---|---|
| | SAMPLING INTERVAL (INITIAL VALUE) | SAMPLING INTERVAL (APPLIED VALUE) | GAUSSIAN FILTER APPLIED AMOUNT (INITIAL VALUE) | GAUSSIAN FILTER APPLIED AMOUNT (APPLIED VALUE) | |
| 1 | 3 | 3 | 60 | 60 | 0.9 |
| 2 | 3 | 3 | 60 | 60 | 0.9 |
| 3 | 3 | 3 | 60 | 65 | 0.8 |
| 4 | 3 | 3 | 62 | 50 | 0.7 |
| 5 | 3 | 3 | 62 | 65 | 0.9 |
| 6 | 3 | 3 | 65 | 65 | 0.9 |
| 7 | 3 | 3 | 65 | 65 | 0.9 |
| 8 | 3 | 5 | 65 | 50 | 0.9 |
| 9 | 5 | 5 | 50 | 50 | 0.9 |
| 10 | 5 | 5 | 50 | 50 | 0.8 |
| | | | | | |
| | | | | | |

## FIG. 7

COEFFICIENT X

701

NEW INITIAL VALUE = CURRENT INITIAL VALUE +
(CURRENT INITIAL VALUE - APPLIED VALUE) * COEFFICIENT X

MUTUAL INFORMATION AMOUNT (NORMALIZED WITH 0 TO1)

## FIG. 8A

801
802

## FIG. 8B

803
804-1
804-2
804-3
804-4
804-5

# FIG. 8C

# FIG. 8D

# FIG. 8E

# FIG. 9

| TARGET SITE | MODEL IMAGE ID |
|---|---|
| LUNG | ID00002 |
| | ID00025 |
| | ID00044 |

*401*

*801*

*802*

*901*

| AREA ID | TYPE ID | ORIGIN | SIZE |
|---|---|---|---|
| 1 | 0 | 20,20,20 | 60,40,20 |

*803*

*804*

| AREA ID | TYPE ID | ORIGIN | SIZE |
|---|---|---|---|
| 1 | 0 | 45,20,20 | 10,50,30 |
| 2 | 0 | 35,20,50 | 10,50,10 |
| 3 | 0 | 25,20,70 | 10,50,10 |
| 4 | 0 | 55,20,50 | 10,50,10 |
| 5 | 0 | 65,20,70 | 10,50,10 |

*902*

*811*  *810*

*812*

| AREA ID | TYPE ID | ORIGIN | SIZE |
|---|---|---|---|
| 1 | 0 | 20,20,20 | 60,40,20 |
| 2 | 1 | 45,20,35 | 40,40,30 |

*903*

# FIG. 10

1001

Y

ORIGIN (0,0,0)

1002

Z    X

x1    y1

z1

ORIGIN OF RECTANGLE
(x0、y0、z0)

# FIG. 11

1103    1104    1101

1102

MUTUAL INFORMATION AMOUNT

NUMBER OF ALIGNMENT PROCESSES

# FIG. 12

1201

ID00003-1

1203

1202

ID00003-2

1204

ID00003

1205

# FIG. 13

# FIG. 14

EP 2 719 325 A1

# FIG. 15

**1301**
HOSPITAL A

**1305**
SERVICE CENTER

**1302**
HOSPITAL B

(IMAGE PATIENT USING EQUIPMENT A IN HOSPITAL B)

**1501**

TRANSFER PARAMETER SET (PSa) REGARDING IMAGING
TECHNIQUE USING EQUIPMENT A AND EQUIPMENT B

| STORE PSa |

TRANSFER PARAMETER SET (PSb) REGARDING
IMAGING TECHNIQUE USING EQUIPMENT A

**1502**

| STORE PSb |

(HOSPITAL B, WHICH HAS NO EQUIPMENT B, INTRODUCES PATIENT TO HOSPITAL A FOR IMAGING WITH EQUIPMENT B)

**1503**

IMAGE B IMAGED BY EQUIPMENT B IS TRANSFERRED

INQUIRE PRESENCE OF PARAMETER SET REGARDING IMAGING
TECHNIQUE USING EQUIPMENT A AND EQUIPMENT B

**1504**

| CONFIRM PRESENCE OF PSa |

AFFIRM PRESENCE

**1505**

**1506**

REQUEST TRANSFER OF PARAMETER SET

TRANSFER PSa

**1507**

| EXECUTE REGISTRATION PROCESS
BETWEEN IMAGE TAKEN BY EQUIPMENT A
IN HOSPITAL B AND IMAGE TAKEN BY
EQUIPMENT B IN HOSPITAL A USING PSa |

**1508**

# FIG. 16

**1403**
IMAGE INTERPRETATION ROOM

**1401**
PARAMETER MANAGEMENT SERVER

*1601*

**1402**
LABORATORY

(IMAGING PATIENT A)

TRANSFER IMAGE A TAKEN

(INTERPRET IMAGE A)

*1602*

TRANSFER PARAMETER OF REGISTRATION PROCESS
EXECUTED IN IMAGE INTERPRETATION ROOM

OPTIMIZE PROCESSING PARAMETER AND
STORE STORE IT AS PARAMETER SET

*1603*

(IMAGE PATIENT A)

TRANSFER IMAGE A TAKEN IN THE PAST

*1604*

CALL UP PARAMETER SET REGARDING IMAGING EQUIPMENT
AND IMAGING TECHNIQUE USED FOR TESTING

*1605*

PARAMETER SET

TRANSFER PARAMETER SET

EXECUTE REGISTRATION PROCESS BETWEEN
IMAGE A AND IMAGE B TAKEN THIS TIME
USING TRANSFERRED PARAMETER SET

IF COMPARISON IS NOT SUCCESSFUL,
CHANGE POSTURE OF PATIENT OF IMAGING
CONDITION AND RE-EXECUTE IMAGING
AND REGISTRATION PROCESS

TRANSFER IMAGE B TAKEN

*1607*

TRANSFER PARAMETER OF REGISTRATION
PROCESS EXECUTED IN LABORATORY

*1606*

**1608**

CALL UP PARAMETER SET REGARDING
IMAGING EQUIPMENT AND IMAGING
TECHNIQUE USED FOR TESTING

OPTIMIZE PROCESSING PARAMETER AND
STORE STORE IT AS PARAMETER SET

READ PARAMETER SET

TRANSFER PARAMETER SET

*1609*

EXECUTE REGISTRATION PROCESS
BETWEEN IMAGE A AND IMAGE B USING
TRANSFERRED PARAMETER SET

(INTERPRET IMAGE A)

# FIG. 17

PREPROCESSING FILTER — GAUSSIAN
APPLIED AMOUNT — 50%
SERIAL PROCESSING STEP — 3pixel
TRUNCATION ERROR — 2pixel

AUTOMATIC PARAMETER SETTING
PARAMETER MODIFICATION
REGISTRATION EXECUTION
IMAGE DATA 2 ONLY
SUPERIMPOSED DISPLAY

BRIGHTNESS
0   50   100
HUE
R   G   B

EP 2 719 325 A1

## INTERNATIONAL SEARCH REPORT

| International application No. |
| --- |
| PCT/JP2012/063097 |

A. CLASSIFICATION OF SUBJECT MATTER
*A61B5/00*(2006.01)i, *A61B5/055*(2006.01)i, *A61B6/00*(2006.01)i, *A61B6/03*
(2006.01)i

According to International Patent Classification (IPC) or to both national classification and IPC

B. FIELDS SEARCHED

Minimum documentation searched (classification system followed by classification symbols)
A61B5/00, A61B5/055, A61B6/00, A61B6/03

Documentation searched other than minimum documentation to the extent that such documents are included in the fields searched
Jitsuyo Shinan Koho          1922-1996   Jitsuyo Shinan Toroku Koho   1996-2012
Kokai Jitsuyo Shinan Koho    1971-2012   Toroku Jitsuyo Shinan Koho   1994-2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y1<br>A | JP 2003-517361 A  (Siemens Corporate Research Inc.),<br>27 May 2003 (27.05.2003),<br>entire text; all drawings<br>& WO 2001/045047 A1      & US 6563941 B1<br>& EP 1238368 A | 1-6,12-15<br>9,17<br>7,8,10,11,16 |
| X<br>Y1<br>A | JP 2007-29502 A  (Fujifilm Corp.),<br>08 February 2007 (08.02.2007),<br>entire text; all drawings<br>& US 2007/0036410 A1      & JP 4541988 B2 | 1-4,12,13,15<br>9,17<br>5-8,10,11,<br>14,16 |
| Y2 | JP 8-103439 A  (Konica Corp.),<br>23 April 1996 (23.04.1996),<br>paragraph [0068]; fig. 5<br>(Family: none) | 9,17 |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>12 June, 2012 (12.06.12) | Date of mailing of the international search report<br>19 June, 2012 (19.06.12) |
| --- | --- |
| Name and mailing address of the ISA/<br>Japanese Patent Office | Authorized officer |
| Facsimile No. | Telephone No. |

Form PCT/ISA/210 (second sheet) (July 2009)

## INTERNATIONAL SEARCH REPORT

International application No.

PCT/JP2012/063097

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
    It is considered that the matter common to the inventions of claims 1-17 is equivalent to the matter set forth in claim 1.
    However, the search revealed that the matter set forth in claim 1 is not novel, since the matter is disclosed in JP 2003-517361 A (Siemens Corporate Research Inc.), 27 May 2003 (27.05.2003), entire text, all drawings and JP 2007-292502 A (Fujifilm Corp.), 08 February 2007 (08.02.2007), entire text, all drawings.
(Continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2012/063097

Continuation of Box No.III of continuation of first sheet(2)

As a result, since the matter set forth in claim 1 does not make a contribution over the prior art, this matter is not a special technical feature within the meaning of PCT Rule 13.2, second sentence.

Consequently, it is obvious that the inventions of claims 1-17 do not comply with the requirement of unity of invention.

Form PCT/ISA/210 (extra sheet) (July 2009)

## REFERENCES CITED IN THE DESCRIPTION

**Patent documents cited in the description**

• JP 2009160045 A

**Non-patent literature cited in the description**

• *Journal of Institute of Electronics, Information and Communication Engineers D-II,* October 2004, vol. J87-D-II (10), 1887-1920